# EUROPEAN PATENT SPECIFICATION

(11) **EP 4 424 161 B1**
(45) Date of publication and mention of the grant of the patent: **30.09.2026**
(21) Application number: 22907659.1
(22) Date of filing: 17.10.2022
(51) Int. Cl.: A01N 1/145, C12N 5/00, G01N 35/00, G01N 35/10, G01N 35/04

(54) **CRYOGENIC TANK APPARATUS**
KRYOGENE TANKVORRICHTUNG
APPAREIL DE TYPE RÉSERVOIR CRYOGÉNIQUE

(30) Priority: 15.12.2021 KR 20210179615; 28.09.2022 KR 20220123061
(43) Date of publication of application: 04.09.2024
(73) Proprietor: Celltrio, Inc., Fremont, California 94539 (US)
(72) Inventor: KIM, Jin-Oh, Seoul 06691 (KR); HAN, Jin Seok, Seoul 05823 (KR)
(74) Representative: BCKIP Part mbB
(86) International application number: PCT/KR2022/015752
(87) International publication number: WO 2023/113188

(56) References cited:
- CN-A- 101 808 608
- CN-A- 110 436 027
- JP-A- 2013 171 757
- JP-A- 2017 049 073
- KR-A- 20170 129 123
- KR-A- 20200 017 946
- KR-B1- 102 168 670
- KR-B1- 102 168 670

## Description

### [Technical Field]

The present invention relates to a cryogenic tank apparatus with a structure for introducing a gas.

### [Background Art]

Cryopreservation refers to a technology that stores biological materials (cells) in a cryogenic state to temporarily stop life activities and then thaw and use the biological materials when needed.

In general, a cryogenic storage refrigerator may enable cryopreservation of biological materials by providing a cryogenic environment controlled to accommodate a plurality of biological materials.

In this regard, as the related art, Korean Patent No. 1423411 discloses a cryogenic storage tank. In general, according to a cryogenic storage tank apparatus, in order for an operator to extract a vial containing a target material or a box containing a plurality of vials from the cryogenic storage tank apparatus, the operator manually withdraws a rack, which accommodates the box containing the plurality of vials, from the storage tank and manually retrieves the box or the vial from the rack. This process makes it inconvenient for the operator to retrieve the vial or the box.

KR 102168670 B1 discloses a cryogenic tank comprising a receiving part having a rotatable frame structure disposed therein and a cover covering the upper portion of the receiving unit. A frame structure for guiding a plurality of racks to be disposed in a preset position may be provided in the receiving portion. The frame structure is rotatable.

### [Disclosure of the Invention]

### [Technical Problem]

The present invention has been made in an effort to solve the problems in the related art, and an object of the present invention is to provide a cryogenic tank apparatus, which is configured to reduce the amount of occurrence of icing when a vial or a box is unloaded.

However, technical problems to be solved by the the present invention are not limited to the aforementioned technical problem, and other technical problems may be present.

### [Technical Solution]

As a technical solution for achieving the above-mentioned object, the present invention provides a cryogenic tank apparatus according to claim 1.

A moving structure according to an embodiment of the present invention may include: a preparation part on which a box unloaded from a cryogenic tank structure is loaded and positioned or the box to be loaded into the cryogenic tank structure is positioned; a first working stage positioned at one side of the preparation part based on an x-axis and configured to mount the box; a second working stage positioned between the first working stage and the preparation part and configured to mount the box; a vial transfer part configured to be movable along a z-axis and movable along the x-axis and a y-axis at a height higher than the preparation part, the first working stage, and the second working stage and including a vial picker configured to grip a vial; and a box transfer part configured to be movable along the z-axis and movable along the x-axis and the y-axis at a height higher than the preparation part, the first working stage, and the second working stage and including a box gripper configured to grip the box.

**In** the moving structure according to the embodiment of the present invention, the first working stage may be used to load the box into the cryogenic tank structure or unload the box from the cryogenic tank structure, and the first working stage and the second working stage may be used to load the vial into the cryogenic tank structure or box unload the vial from the cryogenic tank structure.

**In** the moving structure according to the embodiment of the present invention, the box may have a through port into which the vial is inserted, barcodes may be marked on lower surfaces of the box and the vial, a barcode reader may be provided on the first working stage and configured to read the barcode of the vial or the barcode of the box, and the moving structure may further include a controller configured to perform the loading or unloading of the box into or from the cryogenic tank structure and the loading or unloading of the vial into or from the cryogenic tank structure on the basis of information on the barcode read by the barcode reader and information inputted from the outside.

**In** the moving structure according to the embodiment of the present invention, the vial transfer part may further include a pick-up restriction unit configured to restrict picking-up of a vial adjacent to the vial picked up by the vial picker.

**In** the moving structure according to the embodiment of the present invention, the vial transfer part may further include an external force applying unit configured to apply an external force downward to the vial placed by the vial picker.

**In** the moving structure according to the embodiment of the present invention, the preparation part may be equipped with a cradle on which the box is placed, and the box gripper may grip the box unloaded from the cryogenic tank structure and then move the unloaded box relative to the cradle in a state in which at least a part of a lower surface of the unloaded box is in contact with the cradle so that at least a part of ice on the lower surface of the unloaded box is removed.

The moving structure according to the embodiment of the present invention may further include: a housing configured to surround the preparation part, the first working stage, the second working stage, the vial transfer part, and the box transfer part, in which the housing has a communication port configured to communicate with the cryogenic tank structure and has a door configured to selectively allow the first working stage to communicate with the outside, and in which the first working stage includes a transfer part configured to operate a table, on which the box is placed, and allow the table to protrude to the outside of the housing through the door.

In the moving structure according to the embodiment of the present invention, the box gripper may transfer the box on the first working stage to the preparation part in order to load the box into the cryogenic tank structure, in which in order to load the vial into the cryogenic tank structure, the box, in which the vial to be loaded is to be positioned, is unloaded from the cryogenic tank structure and prepared on the preparation part, the box, which accommodates the vial to be loaded, is prepared on the first working stage, the box gripper picks up the box prepared on the preparation part and positions the box on the second working stage, the vial picker picks up the vial, which is to be loaded, from the box on the first working stage and places the vial in the box positioned on the second working stage, and the box gripper picks up the box positioned on the second working stage and places the box on the preparation part, and in which in order to unload the vial from the cryogenic tank structure, the box, in which the vial to be unloaded is positioned, is unloaded from the cryogenic tank structure and prepared on the preparation part, the box, in which the vial to be unloaded is to be placed, is prepared on the first working stage, the box gripper picks up the box prepared on the preparation part and positions the box on the second working stage, and the vial picker picks up the vial to be unloaded from the box positioned on the second working stage and places the vial in the box on the first working stage.

Further disclosed is a cryogenic tank apparatus, which may include: a tank part having therein a plurality of racks on which boxes are stacked in a vertical direction; a cryogenic tank structure including an inlet/outlet guide part disposed at an upper side of the tank part and configured to guide unloading of the rack from the tank part or loading of the rack into the tank part; a preparation part on which the box unloaded from the inlet/outlet guide part is loaded and positioned or the box to be loaded into the inlet/outlet guide part is positioned; a first working stage positioned at one side of the preparation part based on an x-axis and configured to mount the box; a second working stage positioned between the first working stage and the preparation part and configured to mount the box; a vial transfer part configured to be movable along a z-axis and movable along the x-axis and a y-axis on the preparation part, the first working stage, and the second working stage and including a vial picker configured to grip the vial; and a box transfer part configured to be movable along the z-axis and movable along the x-axis and the y-axis on the preparation part, the first working stage, and the second working stage and including a box gripper configured to grip the box.

In the cryogenic tank apparatus according to the embodiment of the present invention, the tank part or the moving structure may have a lower humidity made by the gas introduced thereinto than a case in which no gas is supplied, such that the amount of occurrence of icing may be reduced.

In the cryogenic tank apparatus according to the present invention, the pipe structure includes a main pipe configured to communicate with the inside of the tank part, a first branch pipe configured to allow the main pipe to communicate with the inlet/outlet guide part part, a second branch pipe configured to allow the main pipe to communicate with the outside, and a valve configured to open or close the second branch pipe.

The valve may be opened to reduce the amount of gas to be introduced into the first branch pipe.

In the cryogenic tank apparatus according to the embodiment of the present invention, an inner diameter of the second branch pipe may be set to be larger than an inner diameter of the first branch pipe so that the amount of gas introduced into the first branch pipe is smaller than the amount of gas introduced into the second branch pipe when the valve is opened.

In the cryogenic tank apparatus according to the embodiment of the present invention, the gas may be nitrogen gas.

In the cryogenic tank apparatus according to the embodiment of the present invention, the tank part may have a hollow portion having a plurality of racks on which the boxes are stacked in the vertical direction, the tank part may maintain an internal temperature to be a preset temperature, a door may be provided in an upper surface of the tank part to allow the rack to enter or exit the tank part, and the inlet/outlet guide part may include a robot part disposed at the upper side of the tank part and configured to open or close the door, the robot part being configured to unload the rack from the hollow portion or load the rack into the hollow portion, and a housing provided at the upper side of the tank part and configured to surround the robot part.

The technical solution is just illustrative but should not be interpreted as being intended to limit the present invention. In addition to the above-mentioned exemplary embodiment, additional exemplary embodiments may be present in the drawings and the detailed description of the present disclosure.

### [Advantageous Effects]

According to the present invention, the vial or the box unloaded from the cryogenic tank structure may be provided to the operator (user) by the moving structure, the vial or the box provided by the operator may be loaded into the cryogenic tank structure by the moving structure, such that the vial and the box may be automatically unloaded from the cryogenic tank structure, or the vial and the box may be automatically loaded into the cryogenic tank structure.

In addition, according to the present invention, at least a part of the ice on the box unloaded from the cryogenic tank structure may be removed, and then the box may be provided to the operator. Further, the vial, which is attached to an adjacent vial by icing after being unloaded from the cryogenic tank structure, may be separated from the adjacent vial, and then the vial may be provided to the operator.

In addition, according to the present invention, at least a part of the gas in the tank part of the cryogenic tank structure may be introduced into the inlet/outlet guide part and the moving structure, thereby at least partially preventing the occurrence of icing after the box is unloaded from the tank part.

### [Description of Drawings]

FIG. 1 is a schematic perspective view of a cryogenic tank apparatus including a moving structure according to an embodiment of the present invention.
FIG. 2 is a schematic perspective view of the moving structure from which a housing according to the embodiment of the present invention is eliminated.
FIG. 3 is a schematic perspective view illustrating the moving structure from which the housing according to the embodiment of the present invention is excluded when viewed at an angle different from an angle in FIG. 2.

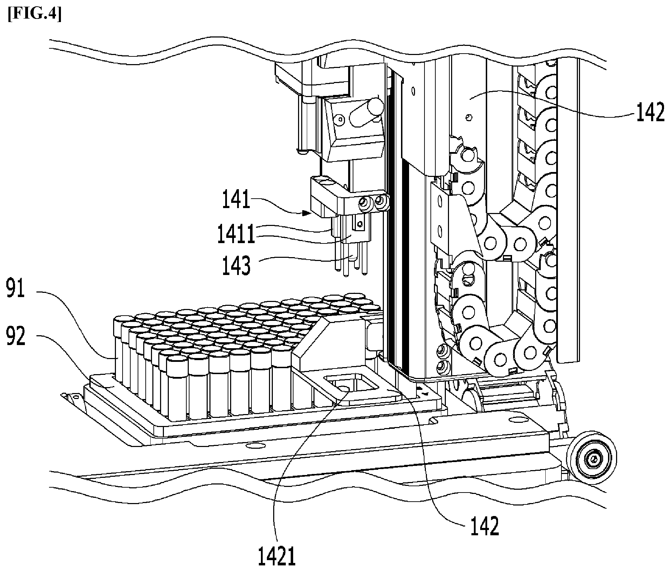

FIG. 4 is a schematic perspective view of a part of a vial transfer part of the moving structure according to the embodiment of the present invention.
FIG. 5 is a schematic perspective view of the cryogenic tank apparatus according to the embodiment of the present invention.
FIG. 6 is a schematic perspective view illustrating the cryogenic tank apparatus according to the embodiment of the present invention in a state in which some components such as the housing of the moving structure and a housing of an inlet/outlet guide part are excluded.
FIG. 7 is a schematic perspective view of the cryogenic tank apparatus according to another embodiment of the present invention.

### [Best Mode]

Hereinafter, embodiments of the present invention will be described in detail with reference to the accompanying drawings so that those with ordinary skill in the art to which the present invention pertains may easily carry out the embodiments. However, the present invention may be implemented in various different ways, and is not limited to the embodiments described herein. A part irrelevant to the description will be omitted in the drawings in order to clearly describe the present invention, and similar constituent elements will be designated by similar reference numerals throughout the specification.

Throughout the specification of the present invention, when one constituent element is referred to as being "connected to" another constituent element, one constituent element can be "directly connected to" the other constituent element, and one constituent element can also be "electrically connected to" the other element with other elements therebetween.

Throughout the specification of the present invention, when one member is disposed "on", "at an upper side of", "at an upper end of", "below", "at a lower side of", or "at a lower end of" another member in the present specification of the present invention, this includes not only a case where one member is brought into contact with another member, but also a case where still another member is present between the two members.

Throughout the specification of the present invention, unless explicitly described to the contrary, the word "comprise" or "include" and variations, such as "comprises", "comprising", "includes" or "including", will be understood to imply the inclusion of stated constituent elements, not the exclusion of any other constituent elements.

The present invention relates to a moving structure and a cryogenic tank apparatus including the same.

First, a moving structure (hereinafter, referred to as 'the present moving structure') 1 according to an embodiment of the present invention will be described.

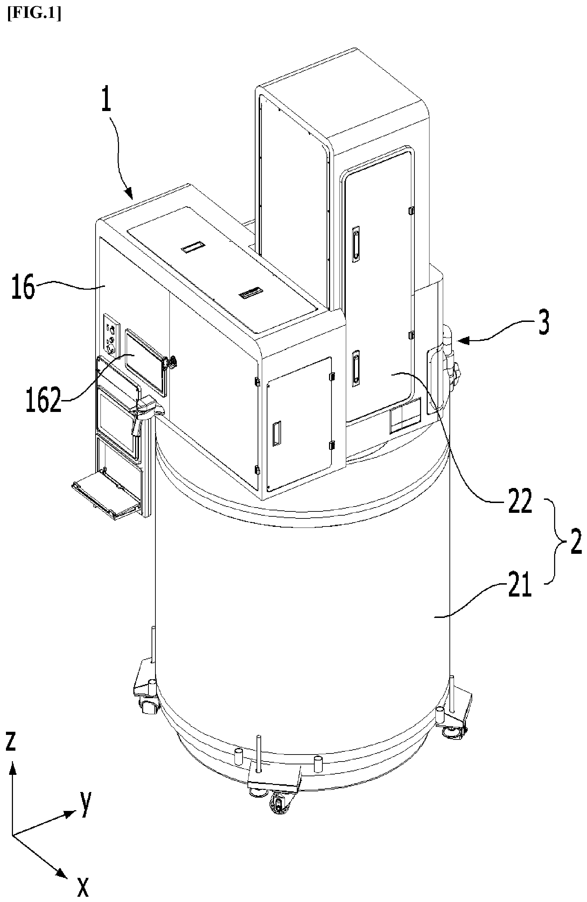

With reference to FIG. 1, the present moving structure 1 is applied to load or unload a vial 91 or a box 92 containing the vials 91 into or from a cryogenic tank structure 2.

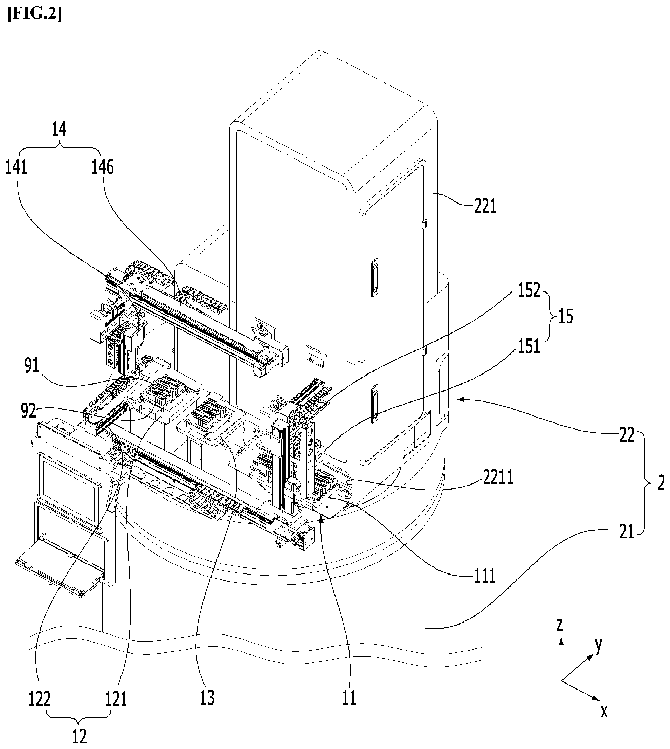

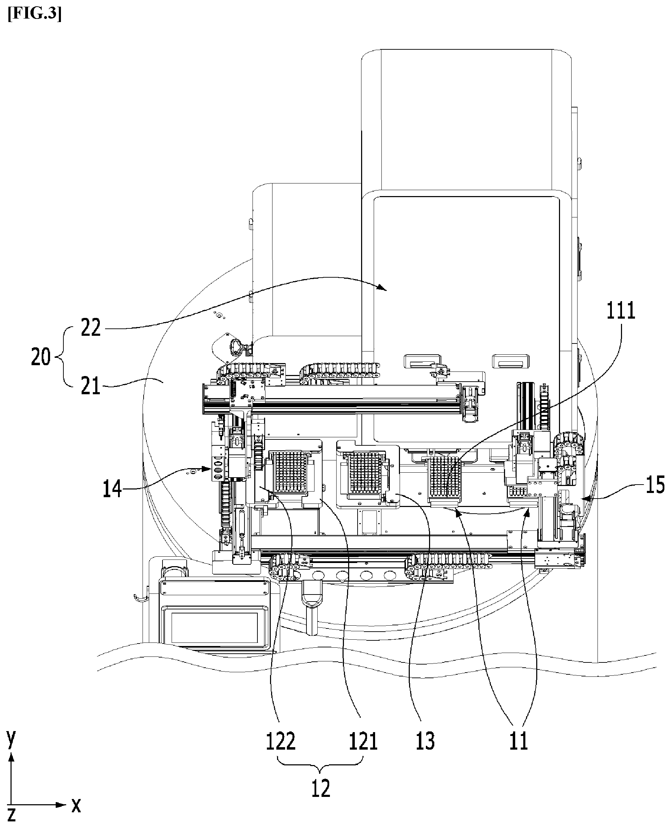

Specifically, with reference to FIGS. 2 and 3, the present moving structure 1 includes a preparation part 11. In the preparation part 11, the box unloaded from the cryogenic tank structure 2 is positioned, or the box loaded into the cryogenic tank structure 2 is positioned.

In addition, with reference to FIGS. 2 and 3, the present moving structure 1 includes a first working stage 12 positioned at one side of the preparation part 11 based on an x-axis, and the box 92 may be mounted on the first working stage 12.

In addition, with reference to FIGS. 2 and 3, the present moving structure 1 includes a second working stage 13 positioned between the first working stage 12 and the preparation part 11, and the box 92 may be mounted on the second working stage 13.

In addition, with reference to FIGS. 2 and 3, the present moving structure 1 includes a vial transfer part 14. The vial transfer part 24 may include a vial picker 141 configured to move along a z-axis, move along the x-axis and y-axis at a height higher than the preparation part 11, the first working stage 12, and the second working stage, and grip the vial 91.

In addition, with reference to FIGS. 2 and 3, the present moving structure 1 includes a box transfer part 15. The box transfer part 15 includes a box gripper 151 configured to move along the z-axis, move along the x-axis and y-axis at a height higher than the preparation part 11, the first working stage 12, and the second working stage 13, and grip the box 92. In addition, the box transfer part 15 may include a gripper transfer part 152 configured to transfer the box gripper 151 along the x-axis, y-axis, and z-axis. In order to guide a transfer of a gripper 151, the gripper transfer part 152 may include three rails respectively extending along the x-axis, y-axis, and z-axis, and a motor configured to transfer the gripper 151 along the three rails.

In addition, the present moving structure 1 may include a housing 16 configured to surround the preparation part 11, the first working stage 12, the second working stage 13, the vial transfer part 14, and the box transfer part 15. The housing 16 may have a communication port 161 configured to communicate with the cryogenic tank structure 2. In addition, the housing 16 may be equipped with a door 162 configured to allow the first working stage 12 to communicate with the outside. The door 162 may be automatically or manually opened or closed.

In addition, the first working stage 12 may include a table 121 on which the box 92 is placed, and a transfer part 122 configured to operate to allow the table 121 to protrude to the outside of the housing 16 through the door 162. For example, the transfer part 122 may move the table 121 along the y-axis. Therefore, when the door 162 is opened, the transfer part 122 moves the table 121 along the y-axis to allow at least a part of the table 121 to protrude to the outside of the housing 16. An operator may retrieve the box 92 on the table 121, retrieve a necessary the vial 91 among the plurality of vials in the box 92, placing the box 92 on the table 121 or placing the vial 91 in the box 92 on the table 121.

The first working stage 12 may be used to load the box 92 into the cryogenic tank structure 2 or unload the box 92 from the cryogenic tank structure 2. In this case, the loading of the box 92 into the cryogenic tank structure 2 may mean a process of loading (moving) the box 92 into the tank structure 2 from the outside of the moving structure 1 or the moving structure 1. In addition, the unloading of the box 92 from the cryogenic tank structure 2 may move a process of unloading (moving) the box 92 from the tank structure 2 to the outside of the moving structure.

In addition, the first working stage 12 and the second working stage 13 may be used to load the vial 91 into the cryogenic tank structure 2 or unload the vial 91 from the cryogenic tank structure 2. In this case, the loading of the vial 91 into the cryogenic tank structure 2 may mean a process of loading (moving) the vial 91 into the tank structure 2 from the outside of the moving structure 1 or the moving structure 1. In addition, the unloading of the vial 91 from the cryogenic tank structure 2 may move a process of unloading (moving) the vial 91 from the tank structure 2 to the outside of the moving structure.

In addition, the box 92 may have a plurality of through ports into which the plurality of vials 91 is inserted. The through ports may be formed in n rows and m columns. The plurality of vials 91 may be respectively inserted into the plurality of through ports, the plurality of through ports may be formed so that the plurality of vials 91 is not withdrawn from the through ports. For example, the through port may have a quadrangular cross-section. One of or both a horizontal length and a vertical length of a quadrangular cross-section of a lower end of the through port may be set to be smaller than an outer diameter of the vial 91.

In addition, barcodes may be marked on a lower surface the box 92 and a lower surface of the vial 91.

Therefore, the barcode on the vial 91 inserted and disposed into the through port of the box 92 may be exposed downward, the barcode on the box 92 may also be exposed downward.

In addition, the first working stage 12 may be equipped with a barcode reader configured to read the barcode of the vial 91 or the barcode of the box 92. For example, the lower surface of the box 92 on which the barcode is formed may be exposed toward a lower side of the table 121, the lower surfaces of the plurality of vials 91 may be exposed downward, and the barcode reader may be provided at the lower side of the table 121.

In addition, the present moving structure 1 may include a controller. On the basis of information about the barcode read by the barcode reader and information inputted from the outside, the controller may load or unload the box 92 into or from the cryogenic tank structure 2 and load or unload the vial 91 into or from the cryogenic tank structure 2.

Specifically, in order to load the box 92 into the cryogenic tank structure 2, the box gripper 151 may transfer the box 92 on the first working stage 12 to the preparation part 11. Specifically, the operator may manually or automatically open the door 162. When the door 162 is opened, the table 121 may move to one side based on the y-axis, such that at least a part of the table 121 may protrude to the outside of the housing 16, and the operator may place the box 92 on the table 121. Thereafter, the table 121 may be moved to the other side based on the y-axis, loaded into the housing 16, and returned to an initial position. At the initial position, the barcode reader may input the barcode of the box 92 or the barcode of the vial 91 disposed in the box 92. The box gripper 151 may move while gripping the box 92 on the table 121 of the first working stage 16 and place the box 92 on the preparation part 11. When the box 92 is placed on the preparation part 11, the box 92 may be loaded into a tank part 21 of the cryogenic tank structure 2. In this case, the box 92 is loaded on the basis of information on the position in the tank part 21 inputted in advance by the operator (information such as which a preset rack the box is placed on among a plurality of racks), or the box 92 is loaded at any position in the tank part 21, and any position may be stored in the controller.

In addition, in order to unload the box 92 from the cryogenic tank structure 2, the box gripper 151 may transfer the box 92 on the preparation part 11 to the first working stage 12. Specifically, when the operator inputs information on the box 92 intended to be unloaded (including barcode information), the box 92 may be unloaded from the cryogenic tank structure 2 and prepared on the preparation part 11 on the basis of the inputted information. The box gripper 151 may grip and transfer the box 92 on the preparation part 11 and place the box 92 on the first working stage 12. The table 121 of the first working stage 12 may move to one side based on the y-axis, such that at least a part of the table 121 may protrude to the outside of the housing 16, and the operator may retrieve the box 92.

In addition, in order to load the vial 91 into the cryogenic tank structure 2, the box 92 in which the vial 91 to be loaded is to be positioned may be unloaded from the cryogenic tank structure 2 and prepared on the preparation part 11, and the box 92 containing the vial 91 may be prepared on the first working stage 12. The box gripper 151 may pick up the box 92 prepared on the preparation part 11 and position the box 92 on the second working stage 13, and the vial picker 141 may pick up the vial 91 loaded from the box 92 of the first working stage 12 and place the vial 91 in the box 92 positioned on the second working stage 13. The box gripper 151 may pick up the box 92 positioned on the second working stage 13 and place the box 92 on the preparation part 11.

Specifically, the operator may manually or automatically open the door 162. When the door 162 is opened, the table 121 may move to one side based on the y-axis, such that at least a part of the table 121 may protrude to the outside of the housing 16, the operator may insert and position the particular vial 91, which is to be loaded, into the box 92 disposed in advance on the table 121 or place the box 92, in which the particular vial 91, on the table 121. At the same time, the box 92, in which the loaded particular vial 91 is to be positioned, positioned in the cryogenic tank structure 2 may be unloaded from the cryogenic tank structure 2 and prepared on the preparation part 11. In addition, when the table 121 is disposed at the initial position, the barcode reader may read the barcode of the particular vial 91 to be loaded, the controller may receive the read information. In addition, on the basis of information inputted by the operator (including information on the position in the cryogenic tank structure 2 at which the particular vial 91 is to be positioned), the controller may perform control to prepare the box 92, in which the particular vial 91 is to be disposed, on the preparation part 11 on the basis of the information inputted by the operator so that the particular vial 91 is loaded into the cryogenic tank structure 2. Thereafter, the box gripper 151 may pick up the box 92 prepared on the preparation part 11 and place (position) the box 92 on the second working stage 13, and the vial picker 141 may pick up the particular vial 91 loaded from the box 92 disposed on the first working stage 12 and place the particular vial 91 in the box 92 positioned on the second working stage 13. The box gripper 151 may pick up the box 92 positioned on the second working stage 13 and place the box 92 on the preparation part 11.

In addition, in order to unload the vial 91 from the cryogenic tank structure 2, the box 92, in which the vial 91 to be unloaded is positioned, may be unloaded from the cryogenic tank structure 2 and prepared on the preparation part 11, and the box 92, in which the vial 91 to be unloaded is to be placed, may be prepared on the first working stage 12. The box gripper 151 may pick up the box 92 prepared on the preparation part 11 and position the box 92 on the second working stage 13, and the vial picker 141 may pick up the vial 91 unloaded from the box 92 positioned on the second working stage 13 and place the vial 91 in the box 92 on the first working stage 12.

Specifically, when the operator inputs information on the particular vial 91 to be unloaded (including one or more pieces of information such as information on barcode name and barcode information), the box 92, in which the particular vial 91 is positioned, may be unloaded from the cryogenic tank structure 2 and prepared on the preparation part 11. In this case, the box 92, in which the particular vial 91 to be unloaded is to be placed, may be prepared on the first working stage 12. The preparation of the box 92, in which the particular vial 91 is to be placed, on the first working stage 12 may be implemented as the box 92 is disposed in advance on the first working stage 12 or the operator opens the door 162 and places the box 92, into which the particular vial 91 is to be inserted, on the first working stage 12. Thereafter, the box gripper 151 may pick up the box 92 prepared on the preparation part 11 and position the box 92 on the second working stage 13, and the vial picker 141 may pick up the vial 91 unloaded from the box 92 positioned on the second working stage 13 and place the vial 91 in the box 92 on the first working stage 12. Thereafter, when the door 162 is opened, the table 121 on the first working stage 12 may move to one side based on the y-axis and protrude to the outside of the housing 16 to provide the vial 91 to the operator.

In addition, the vial transfer part 14 of the present moving structure 1 may include a pick-up restriction unit 142 configured to restrict the picking-up of the adjacent vial 91 adjacent to the vial 91 picked up by the vial picker 141. When the box 92 is unloaded from the cryogenic tank structure 2 in a state in which the vials 91 are contained in the box 92, the vial 91 and the adjacent vial 91 are in a state of being attached to each other by icing. Therefore, when the vial picker 141 picks up the vial 91, the adjacent vial 91 and the vial 91 are attached to each other and picked up together. The pick-up restriction unit 142 may be provided to prevent this situation.

The pick-up restriction unit 142 may be provided at a lower side of the vial picker 141. The pick-up restriction unit 142 may have a through port 1421 formed vertically, and the through port 1421 allows the vial 91 gripped by the vial picker 141 to pass therethrough, but does not allow the adjacent vial 91 to pass therethrough.

In addition, the vial transfer part 14 may include an external force applying unit 143 configured to apply an external force downward to the vial 91 to be placed by the vial picker 141. For example, the vial picker 141 may include a pair of picker units 1411 disposed with the vial picker 141 interposed therebetween and capable of adjusting an interval therebetween. During the gripping operation of the vial picker 141, the interval between the pair of picker units 1411 may be decreased to grip the vial picker 141. The interval may be increased during a releasing operation of the vial picker 141. According to this configuration, the vial 91 may be disposed between the pair of picker units 1411, and the external force applying unit 143 may be positioned to be higher than a height at which the vial 91 is disposed between the pair of picker units 1411, such that the external force applying unit 143 may adjust a length thereof. Therefore, during the process of placing the vial 91, the vial picker 141 may insert the vial 91 into the through port of the box 92. In this process, the external force applying unit 143 may push the vial 91 downward while increasing the length of the external force applying unit 143. Therefore, the vial 91 may be easily inserted into the through port of the box 92 in comparison with a case in which the external force applying unit 143 is not provided. The external force applying unit 143 may include a cylinder.

In addition, the preparation part 11 may be equipped with a cradle 111 on which the box 92 is placed. The box gripper 151 may grip the box 92 unloaded from the cryogenic tank structure 2 and then move the box 92, which is unloaded in a state in which at least a part of lower surface of the unloaded box 92 is in contact with the cradle 111, relative to the cradle 111 in order to remove at least a part of ice on the lower surface of the unloaded box 92. To this end, an upper surface of the cradle 111 may have surface roughness higher than surface roughness of the lower surface of the box 92.

In addition, for reference, the tank part 21 of the cryogenic tank structure 2 may have a plurality of (two) outlets 225. In this case, racks 93 may enter or exit the plurality of outlets 225, respectively. In consideration of the configuration in which the box 92 is loaded or unloaded with respect to the rack 93, the number of communication ports of the housing 16 of the moving structure 1 and the number of communication ports 2211 of a housing 221 of an inlet/outlet guide part 22 of the cryogenic tank structure 2 may be set to corresponding to the number of outlets 225. For example, in case that the number of outlets 225 of the tank part 21 is two, two communication ports of the housing 16 of the moving structure 1 may be provided, two communication ports 2211 of the housing 221 of the inlet/outlet guide part 22 of the cryogenic tank structure 2 may be provided, and the communication ports may communicate with one another. In this case, the preparation part 11 may be equipped with two cradles 111 corresponding (equal) in number to the communication ports.

Hereinafter, a cryogenic tank apparatus (hereinafter, referred to as 'the present first cryogenic tank apparatus') according to the embodiment of the present invention will be described. However, the present first cryogenic tank apparatus includes the present moving structure 1 and shares the identical or corresponding technical features and configurations with the present moving structure 1, the present second cryogenic tank apparatus, and the present third cryogenic tank apparatus that will be described below. Therefore, the repeated description of the present moving structure 1, the present second cryogenic tank apparatus, and the present third cryogenic tank apparatus, which will be described below, will be described briefly or omitted, and the identical or similar components will be assigned with the same reference numerals.

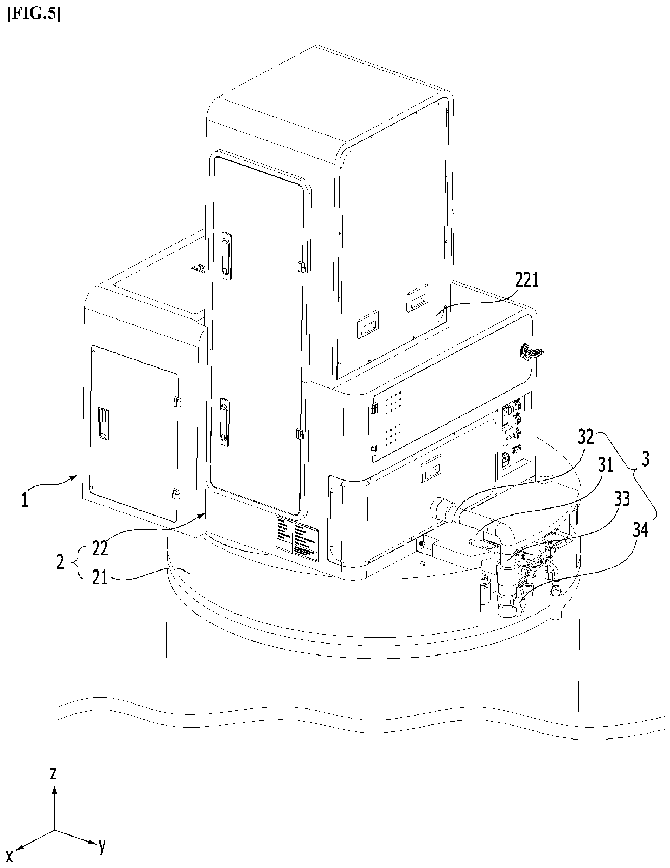

With reference to FIGS. 1 and 5, the present first cryogenic tank apparatus includes the tank part 21 having therein the plurality of racks 93 provided such that the boxes 92 are stacked in a vertical direction.

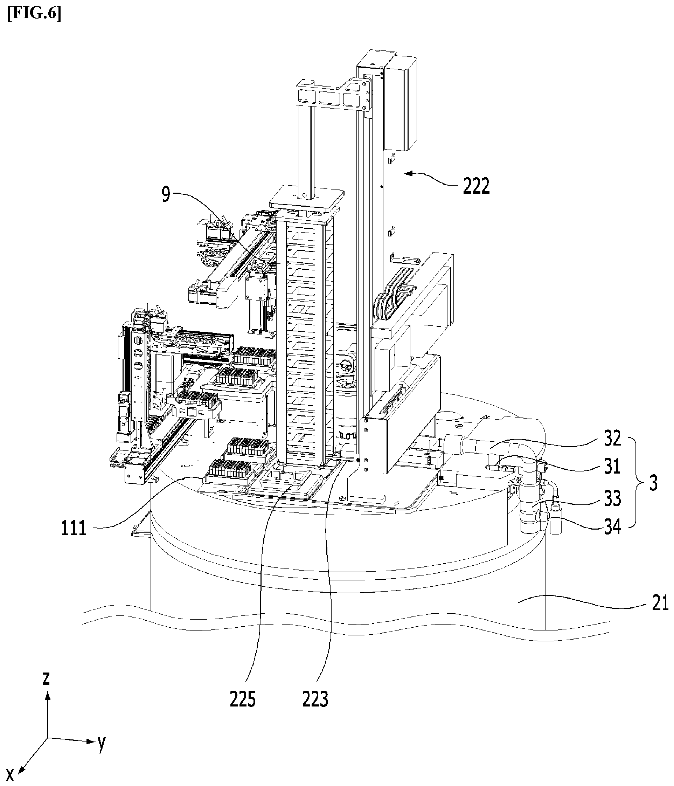

In addition, with reference to FIG. 6, the present first cryogenic tank apparatus includes the inlet/outlet guide part 22 disposed at an upper side of the tank part 21 and configured to guide the loading of the rack 93 or the unloading of the rack with respect to the tank part 21.

In addition, the present first cryogenic tank apparatus includes the preparation part 11. In the preparation part 11, the box unloaded from the cryogenic tank structure 2 is positioned, or the box loaded into the cryogenic tank structure 2 is positioned.

In addition, the present first cryogenic tank apparatus includes the first working stage 12 positioned at one side of the preparation part 11 based on the x-axis, and the box 92 may be mounted on the first working stage 12.

In addition, the present first cryogenic tank apparatus includes the second working stage 13 positioned between the first working stage 12 and the preparation part 11, and the box 92 may be mounted on the second working stage 13.

In addition, the present first cryogenic tank apparatus includes the vial transfer part 14. The vial transfer part 24 may include the vial picker 141 configured to move along the z-axis, move along the x-axis and y-axis at a height higher than the preparation part 11, the first working stage 12, and the second working stage, and grip the vial 91.

In addition, the present first cryogenic tank apparatus includes the box transfer part 15. The box transfer part 15 includes the box gripper 151 configured to move along the z-axis, move along the x-axis and y-axis at a height higher than the preparation part 11, the first working stage 12, and the second working stage 13, and grip the box 92.

Hereinafter, a cryogenic tank apparatus (hereinafter, referred to as 'the present second cryogenic tank apparatus') according to another embodiment of the present invention will be described. However, the present second cryogenic tank apparatus includes the present moving structure 1 and shares the identical or corresponding technical features and configurations with the present moving structure 1, the present first cryogenic tank apparatus, and the present third cryogenic tank apparatus that will be described below. Therefore, the repeated description of the present moving structure 1, the present first cryogenic tank apparatus, and the present third cryogenic tank apparatus, which will be described below, will be described briefly or omitted, and the identical or similar components will be assigned with the same reference numerals.

With reference to FIGS. 1 and 5, the present second cryogenic tank apparatus includes the cryogenic tank structure 2. The cryogenic tank structure 2 includes the tank part 21 having therein the plurality of racks 93 provided such that the boxes 92 are stacked in the vertical direction. The tank part 21 may have therein a hollow portion in which the plurality of racks 93 is accommodated. The plurality of racks 93 may be disposed at intervals in a horizontal direction in the hollow portion. In addition, outlets, through which the racks 93 enter or exit the tank part 21 may be formed in an upper surface of the tank part 21, and doors 225 may be provided in the outlets to open or close the outlets. The tank portion 21 is apparent to those skilled in the art and will not be described in detail.

In addition, with reference to FIG. 5, the cryogenic tank structure 2 includes the inlet/outlet guide part 22 disposed at the upper side of the tank part 21 and configured to guide the loading of the rack 93 or the unloading of the rack with respect to the tank part 21.

Specifically, the inlet/outlet guide part 22 may include the housing 221 disposed at the upper side of the tank part 21. In addition, the inlet/outlet guide part 22 may include a robot part 222. The robot part 222 may be disposed at the upper side of the tank part 21 and open or close the outlet by using the door. In addition, the robot part 222 may unload the rack 93 from the inside of the hollow portion or load the rack 93 into the hollow portion.

In addition, the housing 221 may have a communication port that communicates with the inside of the housing 16 of the present moving structure 1. In addition, the inlet/outlet guide part 22 may include a pusher 223 provided in the housing 221 configured to move the box 92 into the housing 16 (the preparation part 11) of the present moving structure 1 from the rack 93, which is unloaded to the upper side of the tank part 21 through the outlet, through the communication port of the housing 221 of the inlet/outlet guide part 22 and the communication port of the housing 16 of the present moving structure 1.

The inlet/outlet guide part 22 may have a configuration identical or similar to that of a robot device of a cryogenic storage system in the related art. In other words, the inlet/outlet guide part 22 is apparent to those skilled in the art and will not be described in detail.

In addition, the present second cryogenic tank apparatus includes the present moving structure 1. The present moving structure 1 is applied to load or unload the vial 91 or the box 92 containing the vials 91 into or from the cryogenic tank structure 2. That is, the present moving structure 1 may automatically provide the operator with the vial 91 or the rack 92 unloaded from the cryogenic tank structure 2 and automatically load the vial 91 or the rack 92, which is provided by the operator, into the cryogenic tank structure 2.

In addition, with reference to FIGS. 5 and 6, the present second cryogenic tank apparatus includes a pipe structure 3. The pipe structure 3 introduces at least a part of a gas, which maintains a temperature in the tank part 21, into the inlet/outlet guide part 22 or the present moving structure 1. Alternatively, the pipe structure 3 discharges at least a part of the gas, which maintains the temperature in the tank part 21, to the outside.

The gas may be gaseous nitrogen. For example, the tank part 21 may vaporize liquid nitrogen and supply the nitrogen into the hollow portion (interior) to maintain the internal temperature to be a preset temperature (e.g., a low temperature). The pipe structure 3 supplies the gas into the inlet/outlet guide part 22, such that the gas is supplied into the inlet/outlet guide part 22. Because the housing 221 of the inlet/outlet guide part 22 communicates with the housing 16 of the present moving structure 1, at least a part of the gas may also be introduced into the present moving structure 1. Therefore, the gas may be supplied into the present moving structure 1 and the inlet/outlet guide part 22, such that the temperature may be lowered, and a humidity may be lowered, thereby preventing the occurrence of icing.

In other words, the inlet/outlet guide part 22 or the present moving structure 1 has a lower humidity made by the gas introduced thereinto than a case in which no gas is supplied, such that the amount of occurrence of icing may be reduced. For example, icing may occur on the box 92 or the vial 91 when the rack 93 is unloaded from the tank part 21 to the outside of the tank part 21 because of a difference in temperature or humidity between the tank part 21 and the inlet/outlet guide part 22, a difference in temperature or humidity between the tank part 21 and the present moving structure 1, a difference in temperature or humidity between the inlet/outlet guide part 22 and the present moving structure 1, or the like. However, according to the above-mentioned configuration, the occurrence of icing may be reduced because the difference in temperature or humidity between the tank part 21 and the inlet/outlet guide part 22 and the difference in temperature or humidity between the tank part 21 and the present moving structure 1 may be reduced in comparison with the case in which no gas is supplied into the present moving structure 1 and the inlet/outlet guide part 22.

Specifically, with reference to FIGS. 5 and 6, the pipe structure 3 includes a main pipe 31 configured to communicate with the inside of the tank part 21, a first branch pipe 32 configured to allow the main pipe 31 to communicate with the inlet/outlet guide part, a second branch pipe 33 configured to allow the main pipe 31 to communicate with the outside, and a valve 34 configured to open or close the second branch pipe 33.

Therefore, when the valve 34 is turned off, at least a part of the gas in the tank part 21 may be introduced into the inlet/outlet guide part 22 through the main pipe 31 and the first branch pipe 32, and at least a part of the gas introduced into the inlet/outlet guide part 22 may be introduced into the present moving structure 1.

In addition, when the valve 34 is turned on, at least a part of the gas in the tank part 21 may be discharged to the outside through the main pipe 31 and the second branch pipe 33, or at least a part of the gas may be introduced into the inlet/outlet guide part 22 through the main pipe 31 and the first branch pipe 32. That is, when the valve 34 is turned on, the amount of gas introduced into the inlet/outlet guide part 22 may be reduced in comparison with the case in which the valve 34 is turned off.

Therefore, the valve 34 may be opened (turned on) to reduce the amount of gas to be introduced into the first branch pipe 32. For example, when the humidity (or temperature) in the inlet/outlet guide part 22 or the humidity (or temperature) in the present moving structure 1 is equal to or lower than a preset numerical value, it may be necessary to reduce the amount of gas to be introduced into the inlet/outlet guide part 22. In this case, the amount of gas to be introduced into the inlet/outlet guide part 22 may be reduced by opening the valve 34.

In addition, an inner diameter of the second branch pipe 33 may be set to be larger than an inner diameter of the first branch pipe 32 so that the amount of gas introduced into the first branch pipe 32 is smaller than the amount of gas introduced into the second branch pipe 33 when the valve 34 is opened. Therefore, in comparison with the case in which the inner diameter of the first branch pipe 32 is equal to or larger than the inner diameter of the second branch pipe 33, the reduction in amount of gas to be introduced into the inlet/outlet guide part 22 may be more quickly performed, and the temperature adjustment (humidity adjustment) may be quickly and easily performed when the valve 34 is opened.

In addition, with reference to FIG. 6, the inlet/outlet guide part 22 and the upper surface of the tank part 21 may be sealed. In addition, the moving structure 1 and the upper surface of the tank part 21 may be sealed. For example, the sealing may be performed by one or more of an O-ring, foamed silicone, and the like. Therefore, it is possible to prevent the gas introduced into the inlet/outlet guide part 22 and moving structure 1 from leaking at least partially through another portion other than the door 162.

Hereinafter, a cryogenic tank apparatus (hereinafter, referred to as 'the present third cryogenic tank apparatus') according to another embodiment of the present invention will be described. However, the present third cryogenic tank apparatus shares the identical or corresponding technical features and configurations with the present first cryogenic tank apparatus and the present second cryogenic tank apparatus. Therefore, the repeated description of the present moving structure 1, the present first cryogenic tank apparatus, and the present second cryogenic tank apparatus, which will be described below, will be described briefly or omitted, and the identical or similar components will be assigned with the same reference numerals.

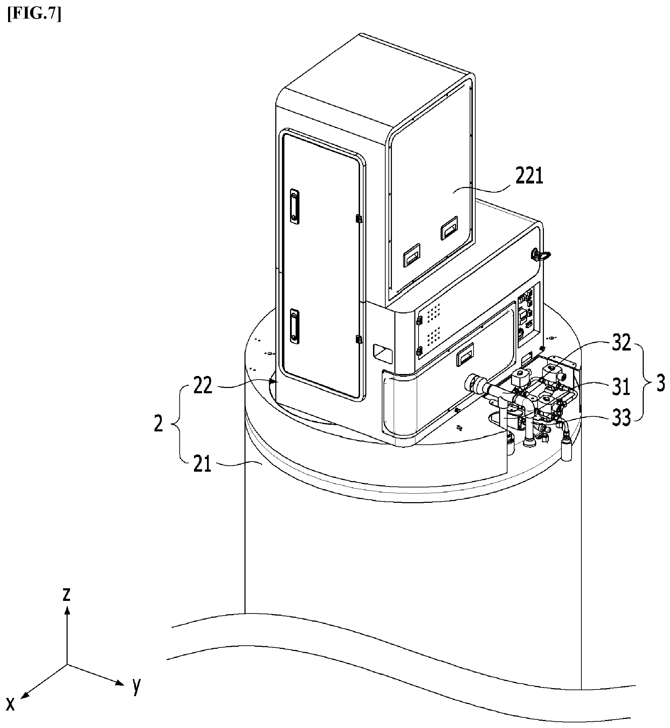

With reference to FIG. 7, the present third cryogenic tank apparatus includes the cryogenic tank structure 2. The cryogenic tank structure 2 includes the tank part 21 having therein the plurality of racks 93 provided such that the boxes 92 are stacked in the vertical direction.

In addition, the cryogenic tank structure 2 includes the inlet/outlet guide part 22 disposed at the upper side of the tank part 21 and configured to guide the loading of the rack 93 or the unloading of the rack with respect to the tank part 21.

In addition, with reference to FIG. 7, the present third cryogenic tank apparatus includes the pipe structure 3 configured to introduce at least a part of the gas, which maintains the temperature in the tank part 21, into the inlet/outlet guide part 22 or discharge the gas to the outside.

With reference to FIG. 7, the pipe structure 3 includes the main pipe 31 configured to communicate with the inside of the tank part 21, the first branch pipe 32 configured to allow the main pipe 31 to communicate with the inside of the tank part 21, the second branch pipe 33 configured to allow the main pipe 31 to communicate with the outside, and a valve (no reference numeral, excluded from FIG. 7) configured to open or close the second branch pipe 33. The valve may be identical to the valve 34 described above with reference to FIGS. 5 and 6.

Therefore, when the valve 34 is turned off, at least a part of the gas in the tank part 21 may be introduced into the inlet/outlet guide part 22 through the main pipe 31 and the first branch pipe 32.

In addition, when the valve 34 is turned on, at least a part of the gas in the tank part 21 may be discharged to the outside through the main pipe 31 and the second branch pipe 33, or at least a part of the gas may be introduced into the inlet/outlet guide part 22 through the main pipe 31 and the first branch pipe 32. That is, when the valve 34 is turned on, the amount of gas introduced into the inlet/outlet guide part 22 may be reduced in comparison with the case in which the valve 34 is turned off.

Therefore, the valve 34 may be opened (turned on) to reduce the amount of gas to be introduced into the first branch pipe 32. For example, when the humidity (or temperature) in the inlet/outlet guide part 22 is equal to or lower than a preset numerical value, it may be necessary to reduce the amount of gas to be introduced into the inlet/outlet guide part 22. In this case, the amount of gas to be introduced into the inlet/outlet guide part 22 may be reduced by opening the valve 34.

In addition, an inner diameter of the second branch pipe 33 may be set to be larger than an inner diameter of the first branch pipe 32 so that the amount of gas introduced into the first branch pipe 32 is smaller than the amount of gas introduced into the second branch pipe 33 when the valve 34 is opened. Therefore, in comparison with the case in which the inner diameter of the first branch pipe 32 is equal to or larger than the inner diameter of the second branch pipe 33, the reduction in amount of gas to be introduced into the inlet/outlet guide part 22 may be more quickly performed, and the temperature adjustment (humidity adjustment) may be quickly and easily performed when the valve 34 is opened.

It will be appreciated that the embodiments of the present invention have been described above for purposes of illustration. Therefore, it should be understood that the above-described embodiments are illustrative in all aspects and do not limit the present invention. For example, each component described as a single type may be carried out in a distributed manner. Likewise, components described as a distributed type can be carried out in a combined type.

The scope of the present application is represented by the claims to be described below rather than the detailed description.

## Claims

1. A cryogenic tank apparatus, comprising:
a cryogenic tank structure (2) including a tank part (21) having therein a plurality of racks (93) on which boxes (92) are stacked in a vertical direction, and an inlet/outlet guide part (22) disposed at an upper side of the tank part (21) and configured to guide unloading or loading of the racks (93) with respect to the tank part (21); and
a pipe structure (3) configured to introduce a gas, which maintains a temperature in the inlet/outlet guide part (22), into the inlet/outlet guide part (22) or discharge the gas to the outside,
wherein the pipe structure (3) includes a main pipe (31) configured to communicate with the inside of the tank part (21), a first branch pipe (32) configured to allow the main pipe (31) to communicate with the inlet/outlet guide part (22), a second branch pipe (33) configured to allow the main pipe (31) to communicate with the outside, and a valve (34) configured to open or close the second branch pipe (33).

2. The cryogenic tank apparatus of claim 1, wherein an inner diameter of the second branch pipe (33) is set to be larger than an inner diameter of the first branch pipe (32) so that the amount of gas introduced into the first branch pipe (32) is smaller than the amount of gas introduced into the second branch pipe (33) when the valve (34) is opened.

3. The cryogenic tank apparatus of any one of the preceding claims, further comprising:
a moving structure (1) applied to load or unload a vial (91) or the box (92) containing the vial (91) into or from the cryogenic tank structure (2), and
wherein the pipe structure (3) is further configured to introduce a gas, which maintains a temperature in the tank part (21), into the moving structure (1).

4. The cryogenic tank apparatus of any one of the preceding claims,
wherein the tank part (21) has a hollow portion having the plurality of racks (93) on which the boxes (92) are stacked in the vertical direction, the tank part (21) is configured to maintain an internal temperature to be a preset temperature, a door (162) is provided in an upper surface of the tank part (21) to allow the racks (93) to enter or exit the tank part (21), and
wherein the inlet/outlet guide part (22) includes a robot part (222) disposed at the upper side of the tank part (21) and configured to open or close the door (162), the robot part (222) being configured to unload the racks (93) from the hollow portion or load the racks (93) into the hollow portion, and a housing (221) provided at the upper side of the tank part (21) and configured to surround the robot part (222).

## Patentansprüche

1. Kryotankvorrichtung, umfassend:
eine Kryotankstruktur (2), die einen Tankteil (21), der darin eine Mehrzahl von Gestellen (93) aufweist, auf denen Boxen (92) in einer vertikalen Richtung gestapelt sind, und einen Ein-/Auslassführungsteil (22) umfasst, der an einer Oberseite des Tankteils (21) angeordnet und dazu eingerichtet ist, das Entladen oder Laden der Gestelle (93) in Bezug auf den Tankteil (21) zu führen; und
eine Rohrstruktur (3), die dazu eingerichtet ist, ein Gas, das eine Temperatur in dem Ein-/Auslassführungsteil (22) aufrechterhält, in den Ein-/Auslassführungsteil (22) einzuleiten oder das Gas nach außen abzuführen,
wobei die Rohrstruktur (3) ein Hauptrohr (31), das dazu eingerichtet ist, mit dem Inneren des Tankteils (21) in Verbindung zu stehen, ein erstes Abzweigrohr (32), das dazu eingerichtet ist, das Hauptrohr (31) mit dem Ein-/Auslassführungsteil (22) in Verbindung zu setzen, ein zweites Abzweigrohr (33), das dazu eingerichtet ist, das Hauptrohr (31) mit der Außenseite in Verbindung zu setzen, und ein Ventil (34) umfasst, das dazu eingerichtet ist, das zweite Abzweigrohr (33) zu öffnen oder zu schließen.

2. Kryotankvorrichtung nach Anspruch 1, wobei ein Innendurchmesser des zweiten Abzweigrohrs (33) so eingestellt ist, dass er größer ist als ein Innendurchmesser des ersten Abzweigrohrs (32), sodass die in das erste Abzweigrohr (32) eingeleitete Gasmenge kleiner ist als die in das zweite Abzweigrohr (33) eingeleitete Gasmenge, wenn das Ventil (34) geöffnet ist.

3. Kryotankvorrichtung nach einem der vorhergehenden Ansprüche, ferner umfassend:
eine Bewegungsstruktur (1), die dazu eingesetzt wird, ein Vial (91) oder die das Vial (91) enthaltende Box (92) in die Kryotankstruktur (2) zu laden oder aus dieser zu entladen, und
wobei die Rohrstruktur (3) ferner dazu eingerichtet ist, ein Gas, das eine Temperatur in dem Tankteil (21) aufrechterhält, in die Bewegungsstruktur (1) einzuleiten.

4. Kryotankvorrichtung nach einem der vorhergehenden Ansprüche,
wobei der Tankteil (21) einen Hohlraum mit der Mehrzahl von Gestellen (93) aufweist, auf denen die Boxen (92) in der vertikalen Richtung gestapelt sind, der Tankteil (21) dazu eingerichtet ist, eine Innentemperatur auf einer voreingestellten Temperatur zu halten, eine Tür (162) in einer oberen Oberfläche des Tankteils (21) vorgesehen ist, um den Gestellen (93) das Eintreten in den Tankteil (21) oder das Austreten aus dem Tankteil (21) zu ermöglichen, und
wobei der Ein-/Auslassführungsteil (22) ein Roboterteil (222), das an der Oberseite des Tankteils (21) angeordnet und dazu eingerichtet ist, die Tür (162) zu öffnen oder zu schließen, wobei das Roboterteil (222) dazu eingerichtet ist, die Gestelle (93) aus dem Hohlraum zu entladen oder die Gestelle (93) in den Hohlraum zu laden, und ein Gehäuse (221) umfasst, das an der Oberseite des Tankteils (21) vorgesehen und dazu eingerichtet ist, das Roboterteil (222) zu umgeben.

## Revendications

1. Dispositif de réservoir cryogénique, comprenant :
une structure de réservoir cryogénique (2) présentant une partie de réservoir (21) contenant une pluralité d'étagères (93) sur lesquelles des boîtes (92) sont empilées dans la direction verticale, et une partie de guidage d'entrée/sortie (22) disposée sur un côté supérieur de la partie de réservoir (21) et prévue pour guider le chargement des étagères (93) dans la partie de réservoir (21) ou leur déchargement hors de celle-ci ; et
une structure de conduite (3) prévue pour introduire dans la partie de guidage d'entrée/sortie (22) un gaz maintenant une température dans ladite partie de guidage d'entrée/sortie (22), ou pour évacuer le gaz vers l'extérieur,
où la structure de conduite (3) comprend une conduite principale (31) prévue pour communiquer avec l'intérieur de la partie de réservoir (21), une première conduite de dérivation (32) prévue pour permettre à la conduite principale (31) de communiquer avec la partie de guidage d'entrée/sortie (22), une deuxième conduite de dérivation (33) prévue pour permettre à la conduite principale (31) de communiquer avec l'extérieur, et une vanne (34) prévue pour ouvrir ou fermer la deuxième conduite de dérivation (33).

2. Dispositif de réservoir cryogénique selon la revendication 1, où le diamètre intérieur de la deuxième conduite de dérivation (33) est défini de manière à être supérieur au diamètre intérieur de la première conduite de dérivation (32), de sorte que la quantité de gaz introduite dans la première conduite de dérivation (32) est inférieure à la quantité de gaz introduite dans la deuxième conduite de dérivation (33) quand la vanne (34) est ouverte.

3. Dispositif de réservoir cryogénique selon l'une des revendications précédentes, comprenant en outre :
une structure mobile (1) appliquée pour le chargement d'un flacon (91) ou de la boîte (92) contenant le flacon (91) dans la structure de réservoir cryogénique (2), ou leur déchargement hors de celle-ci, et
où la structure de conduite (3) est en outre prévue pour introduire dans la structure mobile (1) un gaz maintenant une température dans la partie de réservoir (21).

4. Dispositif de réservoir cryogénique selon l'une des revendications précédentes, où la partie de réservoir (21) présente une partie creuse comportant la pluralité d'étagères (93) sur lesquelles les boîtes (92) sont empilées dans la direction verticale, ladite partie de réservoir (21) est prévue pour maintenir (21) une température intérieure à une température prédéfinie, une porte (162) est disposée dans une surface supérieure de la partie de réservoir (2l) pour permettre aux étagères (93) d'entrer ou de sortir de la partie de réservoir (21), et où la partie de guidage d'entrée/sortie (22) comprend une partie de robot (222) disposée sur un côté supérieur de la partie de réservoir (21) et prévue pour ouvrir ou fermer la porte (162), ladite partie de robot (222) étant prévue pour décharger les étagères (93) de la partie creuse ou pour charger les étagères (93) dans la partie creuse, et une enceinte (221) disposée sur un côté supérieur de la partie de réservoir (21) et prévue pour entourer la partie de robot (222).
